# EUROPÄISCHE PATENTANMELDUNG

(11) **EP 4 748 368 A1**
(43) Veröffentlichungstag der Anmeldung: **27.05.2026**
(21) Anmeldenummer: 24215437.5
(22) Anmeldetag: 26.11.2024
(51) Int. Cl.: A61K 8/43, A61K 8/88, A61Q 11/00, A61K 8/49

(54) **ZUSAMMENSETZUNG FÜR MUNDHYGIENEPRODUKTE ENTHALTEND N-CETYLPYRIDINIUMCHLORID, EIN FLAVONOID UND EIN POLYMER**

(71) Anmelder: Curaden AG, 6010 Kriens (CH)
(72) Erfinder: STUDER, Daniel, 6072 Sachseln (CH); SCIOTTI, Michel-Angelo, 5028 Ueken (CH)
(74) Vertreter: Keller Schneider Patent- und Markenanwälte AG

(57) **Zusammenfassung**

Eine Zusammensetzung, insbesondere zur Prophylaxe und/oder Behandlung von Karies, Gingivitis, Halitosis und/oder Arteriosklerose, umfasst N-Cetylpyridiniumchlorid, mindestens ein Flavonoid, mindestens ein Polymer, im Besonderen ein Homopolymer, umfassend mindestens ein Monomer mit einer polaren funktionellen Gruppe, insbesondere Polylysin, und optional Chlorhexidin, wobei das Gewichtsverhältnis zwischen Chlorhexidin und N-Cetylpyridiniumchlorid zwischen 0 bis 7, insbesondere zwischen 0.01 bis 6, speziell zwischen 0.1 bis 5 im Speziellen zwischen 0.2 bis 4, beispielsweise zwischen 0.4 bis 3.2, liegt.

## Beschreibung

### Technisches Gebiet

Die Erfindung betrifft eine Zusammensetzung, insbesondere zur Prophylaxe und/oder Behandlung von Karies, Gingivitis, Halitosis und/oder Arteriosklerose. Weiter betrifft die Erfindung Mundwasser, Zahnpasta, Zahnpflegekaugummis, Tabletten, Zahnseide und/oder Zahnreinigungsgel umfassend die Zusammensetzung, sowie die Zusammensetzung zur Verwendung bei der Prophylaxe und/oder Behandlung von Karies Gingivitis, Halitosis und/oder Arteriosklerose und die Zusammensetzung zur Verwendung als Arzneimittel.

### Stand der Technik

Die Mundhygiene ist ein essenzieller Bestandteil der täglichen Gesundheitsroutine und zahlreiche Produkte stehen zur Verfügung, um die Mundgesundheit zu unterstützen. Zahnpasta, Mundspülungen und Zahnseide gehören zu den bekanntesten Hilfsmitteln, die versprechen, Karies, Gingivitis und Halitosis effektiv zu verhindern. Besonders im Fokus stehen dabei Inhaltsstoffe wie Fluorid, das gezielt gegen den Kariesverursacher *Streptococcus mutans (S. mutans)* wirken soll.

Die WO 99/18999 (Pharmaceutical Biotechnologies, Inc.) betrifft eine Formulierung hauptsächlich zur Prophylaxe von Plaque und zur Vermeidung von Parodontalkrankheiten wie z.B. Karies, indem die Retentionszeit entsprechender Wirkstoffe in der Mundhöhle erhöht wird. Als Ursache für diese Krankheiten sei u.a. die Bakterienart S. *mutans* verantwortlich. Die Formulierung umfasst Polysaccharid abbauende Enzyme sowie an die Enzyme gekoppelte Ankermoleküle, was in Kombination in einem Enzym-Ankermolekül-Komplex resultiert. Durch die Ankermoleküle kann der Enzym-Ankermolekül-Komplex besser in der Mundhöhle haften und das Enzym länger seine Wirkung entfalten kann. Der Enzym-Ankermolekül-Komplex setzt sich an der Seite des Ankermoleküls z.B. an der vorhandenen Plaque fest, wodurch auch potenzielle Substratstellen zur Anheftung von pathogenen Bakterienkolonien reduziert werden. Als bevorzugte Enzyme werden bspw. Esterasen oder Enzyme zur Spaltung von glykolytischen Bindungen, Etherbindungen oder Kohlenstoff-Stickstoff-Bindungen eingesetzt. Die Formulierung kann z.B. für Mundspülungen eingesetzt werden. Beispielhaft erwähnt die WO 99/18999 zudem als Ankermoleküle Proteine, Proteinfragmente und Polypeptide, wie z.B. Lys-Lys-Glu-Lys-Lys.

In der EP 0 737 470 A1 (Quest International B.V.) geht es um eine Anti-Plaque-Formulierung, in welcher der Anti-Plaque-Wirkstoff in kolloidaler Form und von einem Polymer ummantelt vorliegt. Das Polymer kann Antikörper, Kohlenhydrat- oder Peptidstrukturen aufweisen, die von Plaque produzierenden Bakterien erkannt werden, womit der Wirkstoff, z.B. Zinkoxid, zielgerichtet zu den Bakterien transportiert wird. Bei sauren pH-Werten können im Anschluss z.B. Zink-Ionen freigesetzt werden. Beispielhaft erwähnt die EP 0 737 470 A1 zudem, dass optional antibakterielle Mittel, wie z.B. Chlorhexidin oder Cetylpyridinium Chloride (CPC), eingesetzt werden können.

Die WO 99/20239 (Novo Nordisk A/S) beschreibt eine Zusammensetzung zur Verhinderung oder Behandlung von Plaque, u. a. ausgelöst durch *S*. *mutans.* Dazu werden Stärkehydrolisierende und/oder Stärke-modifizierende Enzyme eingesetzt. Die letztere Gruppe umfasst z.B. CGTAse, Mutanase und/oder Dextranase. Die Zusammensetzung kann Bestandteil einer Zahnpasta oder einer Mundspülung sein und Zusatzstoffe wie bspw. Tenside, Süssungsmittel, Geschmacksstoffe oder antibakterielle Wirkstoffe, wie z.B. Chlorhexidin oder Zink-Ionen aufweisen.

Die bekannten Mundhygieneprodukte haben sich jedoch als unbefriedigend erwiesen. Oftmals können sie zwar kurzfristige Frische und ein sauberes Gefühl bieten, erreichen jedoch nicht immer die gewünschte Langzeitwirkung. In vielen Fällen können chemische Inhaltsstoffe zu Irritationen führen, und die Nutzung von Mundhygieneprodukten mit unzureichender antibakterieller Wirkung kann sogar das Risiko für Zahnprobleme erhöhen. Darüber hinaus können antibakterielle Wirkstoffe, die in einigen Mundhygieneprodukten enthalten sind, zwar kurzfristig die Bakterienzahlen reduzieren, ihre langfristige Wirksamkeit ist jedoch oft fraglich.

Es besteht daher ein Bedarf an verbesserten Lösungen.

### Darstellung der Erfindung

Aufgabe der Erfindung ist es daher, eine Lösung bereitzustellen, welche die im Stand der Technik vorhandenen Nachteile zumindest verringert. Insbesondere ist es Aufgabe der Erfindung, eine Zusammensetzung bereitzustellen, welche das Wachstum von schädlichen Mikroorganismen im Mundraum hemmt, das Speichelbiom schont und eine Langzeitwirkung aufweist. Ferner ist es Aufgabe der Erfindung, Verwendungen der Zusammensetzung bereitzustellen.

Diese Aufgaben werden durch die Merkmale des unabhängigen Anspruchs 1 und der unabhängigen Ansprüche 14 und 15 gelöst.

Die Erfindung betrifft demnach eine Zusammensetzung, insbesondere zur Prophylaxe und/oder Behandlung von Karies, Gingivitis, Halitosis und/oder Arteriosklerose, umfassend
(i) N-Cetylpyridiniumchlorid,
(ii) mindestens ein Flavonoid,
(iii) mindestens ein Polymer, im Besonderen ein Homopolymer, umfassend mindestens ein Monomer mit einer polaren funktionellen Gruppe, insbesondere Polylysin,
(iv) und optional Chlorhexidin,
(v) wobei das Gewichtsverhältnis zwischen Chlorhexidin und N-Cetylpyridiniumchlorid zwischen 0 bis 7, insbesondere zwischen 0.01 bis 6, speziell zwischen 0.1 bis 5 im Speziellen zwischen 0.2 bis 4, beispielsweise zwischen 0.4 bis 3.2, liegt.

"Karies", auch bekannt als Zahnfäule, ist eine fortschreitende Erkrankung der Zähne, die durch die Zerstörung von Zahnhartsubstanz verursacht wird. Sie entsteht, wenn Bakterien in der Mundflora Zucker und andere Kohlenhydrate fermentieren, wodurch Säuren produziert werden. Diese Säuren greifen den Zahnschmelz an und führen zu Demineralisation und letztendlich zu Löchern (Kariesstellen) im Zahn. Unbehandelt kann Karies zu Schmerzen, Entzündungen und sogar zum Verlust von Zähnen führen.

"Gingivitis" ist eine Entzündung des Zahnfleisches, die meist durch eine Ansammlung von Plaque (Bakterienbelag) verursacht wird. Sie ist die früheste Form der Parodontalerkrankung und äußert sich typischerweise durch Symptome wie Rötung, Schwellung und Blutung des Zahnfleisches, insbesondere beim Zähneputzen oder der Verwendung von Zahnseide. Gingivitis ist in der Regel reversibel, wenn sie frühzeitig behandelt wird, vor allem durch eine verbesserte Mundhygiene. Unbehandelt kann sie jedoch zu schwerwiegenderen Formen der Parodontalerkrankung führen, wie Parodontitis.

"Halitosis", umgangssprachlich auch als Mundgeruch bezeichnet, ist ein unangenehmer Geruch aus dem Mund, der durch verschiedene Faktoren verursacht werden kann. Häufige Ursachen sind Bakterien im Mundraum, die Speisereste zersetzen, unzureichende Mundhygiene, bestimmte Nahrungsmittel (wie Knoblauch oder Zwiebeln), Rauchen, trockener Mund oder zugrundeliegende gesundheitliche Probleme. Halitosis kann sowohl vorübergehend als auch chronisch sein. Eine gute Mundhygiene und regelmäßige zahnärztliche Kontrollen können helfen, Halitosis zu verhindern oder zu behandeln.

"Arteriosklerose" ist eine Erkrankung, bei der sich die Arterienwände verdicken und verhärten, was zu einer Einschränkung des Blutflusses führt. Diese Veränderungen entstehen meist durch Ablagerungen von Fett, Cholesterin, Kalzium und anderen Substanzen, die Plaque bilden. Arteriosklerose kann verschiedene Blutgefäße im Körper betreffen und ist ein wichtiger Risikofaktor für Herz-Kreislauf-Erkrankungen, wie Herzinfarkt und Schlaganfall.

"N-Cetylpyridiniumchlorid" (CPC) ist eine chemische Verbindung, die u.a. als Antiseptikum eingesetzt wird. Es wird häufig in Mundspülungen, Zahnpasten und anderen oralen Hygieneprodukten verwendet, um die Mundflora zu regulieren und bakterielles Wachstum zu hemmen. CPC wirkt antibakteriell und kann helfen, Plaque und Mundgeruch zu reduzieren.

"Flavonoide" sind eine große Gruppe von sekundären Pflanzenstoffen, die in vielen Obst- und Gemüsesorten vorkommen. Sie gehören zur Familie der Polyphenole und sind für die Farbe, den Geschmack und den Duft von Pflanzen verantwortlich. Flavonoide besitzen antioxidative Eigenschaften und können helfen, Zellschäden durch freie Radikale zu reduzieren. Sie sind auch für ihre potenziellen gesundheitlichen Vorteile bekannt, darunter die Unterstützung des Herz-Kreislauf-Systems, die Reduzierung von Entzündungen und die Verbesserung der Immunfunktion.

Durch die Reduzierung von oxidativem Stress und Entzündungen können Flavonoide die Endothelfunktion verbessern und die Bildung von atherosklerotischen Plaques hemmen. Dies kann das Risiko einer Arteriosklerose zumindest reduzieren.

Mit einer "polaren funktionellen Gruppe" ist eine funktionelle Gruppe in einem Molekül gemeint, die durch eine ungleiche Verteilung der Elektronen zwischen den Atomen in der Gruppe eine Ladungsverteilung aufweist. Dies führt zu einer partiellen positiven und einer partiellen negativen Ladung innerhalb der Gruppe. Beispiele einer polaren funktionellen Gruppe sind bspw. die Hydroxylgruppe (-OH), Carbonylgruppe (C=O), Aminogruppe (-NH₂), Carboxylgruppe (-COOH), Nitrogruppe (-NO₂), Sulfhydrylgruppe (-SH), Amidgruppe (-CONH₂), Phosphatgruppe (-PO₄³⁻), Peroxidgruppe (-O-O-) und/oder die Cyanogruppe (-CN).

Insbesondere weisen die Monomer des mindestens einen Polymers eine oder mehrere der oben genannten polaren funktionellen Gruppe im nicht-polymerisierten Monomer vor und/oder die oben genannten polaren funktionellen Gruppe sind Bestandteil des Polymers, wobei Sie z.B. durch die Polymerisation der Monomere gebildet wurden.

Im Speziellen beinhaltet das mindestens ein Monomer mit einer polaren funktionellen Gruppe eine Aminosäure, im Speziellen eine Aminosäure welche in unpolymerisiertem Zustand eine zwei Amingruppen und eine Carboxygruppe aufweisst. Ganz speziell bevorzugt ist das mindestens ein Monomer mit einer polaren funktionellen Gruppe Lysin.

Gemäss eines vorteilhaften Ausführungsform basiert das mindestens eine Polymer auf polymerisierten Aminosäuren. Dies bedeutet, dass mindestens 50%, im Speziellem mindesten 75%, beispielsweise mindestens 90%, ganz speziell mindestens 99% oder 100%, aller Monomere im Polymer Aminosäuren bzw. polymerisierte Aminosäuren sind.

"Homopolymere" sind Polymere, die nur aus einer Art von Monomer aufgebaut sind. Das bedeutet, dass sich die wiederholenden Einheiten im Polymer aus denselben Molekülen zusammensetzen. Homopolymere haben oft relativ gleichmäßige physikalische und chemische Eigenschaften, da sie eine einheitliche Struktur aufweisen. Beispiele für Homopolymere sind: Polyethylen (PE), welches nur aus Ethylen-Monomeren besteht und Polypropylen (PP), welches nur aus Propylen-Monomeren besteht.

Einige Homopolymere, z.B. Polyvinylalkohol (PVA), Polyvinylpyrrolidon (PVP), Polyacrylsäure (PAA) und/oder Polyethylenglykol (PEG), haben den Vorteil, dass sie die Viskosität von Mundhygieneprodukten verbessern können, was zu einer angenehmen und stabilen Konsistenz führt. Zudem haben einige Homopolymere, z.B. PVA, PVP und/oder PAA, die Fähigkeit, an Zahnoberflächen zu haften, was die Wirksamkeit von Mundhygieneprodukten verlängern kann. Sie weisen zudem antimikrobielle Eigenschaften auf und haben die Fähigkeit Wasser zu binden, was besonders bei einem trockenen Mund vorteilhaft sein kann. Einige Homopolymere, z.B. PVA, PVP und/oder PAA, können zudem die kontrollierte Freisetzung von Wirkstoffen wie z.B. Fluorid oder anderen Substanzen ermöglichen, was zu einer verlängerten Wirkungsdauer führen und die Effektivität von Mundhygieneprodukten verbessern kann.

"Polylysin" ist im Allgemeinen ein Homopolymer, das aus wiederholten Einheiten der Aminosäure Lysin besteht. Es setzt sich ausschließlich aus Lysin-Monomeren zusammen, die durch Peptidbindungen verknüpft sind. Polylysin ist positiv geladen, da die Seitenketten der Lysin-Einheiten Aminogruppen enthalten, die bei physiologischem pH protoniert sind. Es wird häufig in biologischen Anwendungen verwendet, unter anderem in der Zellkultur, da es die Zelladhäsion unterstützt.

"Chlorhexidin" ist ein antiseptisches und desinfizierendes Mittel, das häufig zur Abtötung von Bakterien, Pilzen und einigen Viren eingesetzt wird. Es wirkt durch die Zerstörung der Zellmembranen von Mikroorganismen und verhindert dadurch deren Wachstum und Vermehrung. Chlorhexidin wird in verschiedenen medizinischen und kosmetischen Anwendungen als Konservierungsmittel verwendet, wie z.B. in Mundspülungen, Wunddesinfektion und zur Hautdesinfektion vor chirurgischen Eingriffen. Es ist bekannt für seine langanhaltende Wirkung und wird oft in der Zahnmedizin zur Reduzierung von Plaque und Zahnfleischentzündungen verwendet.

Ein "Speichelbiom" bezeichnet die Gesamtheit der im Speichel vorkommenden mikrobiellen Gemeinschaften und deren biologischen Bestandteile. Dazu gehören Bakterien, Viren, Pilze, Proteine, Enzyme und andere Moleküle, die im Speichel nachweisbar sind. Das Speichelbiom spielt eine wichtige Rolle bei der Mundgesundheit und kann Hinweise auf den Gesundheitszustand des gesamten Körpers geben, da es mit verschiedenen Erkrankungen, wie Zahnfleischerkrankungen, Karies oder sogar systemischen Erkrankungen, in Verbindung gebracht werden kann.

Überraschenderweise hat sich gezeigt, dass die erfindungsgemässe Zusammensetzung zu einer wirksameren Hemmung des Wachstums von *S*. *mutans* führt, das Speichelbiom weniger beeinträchtigt und eine länger anhaltende Wirkung aufweist als bekannte Zusammensetzungen.

Erfindungsgemässe Zusammensetzungen zeichnen sich z.B. dadurch aus, dass eine hemmende Wirkung in Bezug auf das Wachstum von *S*. *mutans* schon bei höheren Verdünnungen erzielt wird, verglichen mit Zusammensetzungen aus dem Stand der Technik. Ein weiterer Vorteil ist, dass die erfindungsgemässen Zusammensetzungen das Speichelbiom erst ab einer niedrigeren Verdünnung beeinträchtigt, als es aus dem Stand der Technik bekannt ist.

Durch die oben genannten Vorteile braucht es eine geringere Menge der Zusammensetzung, um eine Hemmung von *S*. *mutans* zu bewirken, als es im Stand der Technik bekannt ist. Dies führt einerseits zu einer geringeren Zuführung von chemischen Substanzen in den Körper bei gleichbleibender Effektivität der Mundhygieneprodukte und andererseits zu einem geringeren Verbrauch von Inhaltsstoffen für die Herstellung der erfindungsgemässen Zusammensetzung. Letzteres schont die Umwelt und reduziert die Herstellungskosten.

Zudem ist ein weiterer Vorteil, dass eine höhere Menge der Zusammensetzung nötig ist, um das Speichelbiom zu beeinträchtigen, als im bekannten Stand der Technik. Dies unterstützt die Schonung des Speichelbioms, sodass eine Beeinträchtigung erst ab grösseren Mengen der Zusammensetzung beobachtet werden kann.

Ein beeinträchtigtes Speichelbiom bedeutet, dass das natürliche Gleichgewicht der Mikroorganismen und biochemischen Bestandteile im Speichel gestört ist. Ein gesundes Speichelbiom besteht aus einer Vielzahl von Bakterien, Pilzen, Viren und anderen Mikroben, die in einem harmonischen Gleichgewicht zusammenleben und zur Mundgesundheit beitragen. Eine Beeinträchtigung kann zu folgenden Folgen führen:
- Erhöhtes Risiko für Mundkrankheiten: Ein Ungleichgewicht, wie eine Zunahme krankheitserregender Bakterien, kann zu Zahnfleischentzündungen (Gingivitis), Karies, Parodontitis oder Halitosis führen;
- Verringerte Schutzfunktion: Der Speichel enthält antimikrobielle Substanzen, die dazu beitragen, schädliche Mikroben zu kontrollieren. Eine Störung im Speichelbiom kann diese Schutzfunktion beeinträchtigen und Infektionen begünstigen;
- Mögliche systemische Auswirkungen: Da das Speichelbiom mit dem gesamten Körper verbunden ist, kann eine Störung auch Hinweise auf systemische Erkrankungen wie Diabetes oder Herzerkrankungen geben.

Die erfindungsgemässe Zusammensetzung hat damit den Vorteil, dass sie Mundkrankheiten mindestens reduzieren, insbesondere verhindern, und das Speichelbiom schützen kann. Zudem weist sie eine hohe Remanenz auf.

"Remanenz" bezeichnet eine Nachwirkung eines Zustands über einen längeren Zeitraum, auch nachdem der direkte Einfluss verschwunden ist. Vorliegend bezieht sich Remanenz demnach auf die Langzeitwirkung der Zusammensetzung, die noch besteht, nachdem die Zusammensetzung in den Mundraum eingebracht wurde, z.B. durch eine Mundspülung oder eine Zahnpasta.

Mit einer "Verdünnung" ist die Verringerung der Konzentration der Zusammensetzung durch die Zugabe von einem Verdünnungsmittel wie z.B. Wasser und/oder einem anderen Lösungsmittel gemeint. Bei einer "höheren Verdünnung" ist eine Zusammensetzung stärker verdünnt, bei einer "tieferen Verdünnung" ist eine Zusammensetzung schwächer verdünnt.

Ohne an die Theorie gebunden zu sein wird zudem davon ausgegangen, dass sich die erfindungsgemässe Zusammensetzung positiv auf die Prophylaxe und/oder Behandlung von Arteriosklerose auswirken kann. Da chronische Entzündungen eine wichtige Rolle bei der Entstehung von Arteriosklerose spielen, könnten die entzündungshemmenden Eigenschaften der erfindungsgemässen Zusammensetzung indirekt helfen, das Fortschreiten der Krankheit zu verlangsamen.

Weiter gibt es, ebenfalls ohne an die Theorie gebunden zu sein, Hinweise darauf, dass ein Zusammenhang zwischen oralen Bakterien und Arteriosklerose bestehen könnte. Orale Bakterien könnten durch den Blutkreislauf in die Arterien gelangen und dort entzündliche Prozesse auslösen, die die Bildung atherosklerotischer Plaques fördern. Durch die Reduktion von schädlichen oralen Bakterien, insbesondere durch die erfindungsgemässe Zusammensetzung, könnte somit indirekt das Risiko solcher Entzündungen, und damit eine Arteriosklerose, zumindest reduziert werden.

Versuche haben gezeigt, dass die Komponenten der erfindungsgemässen Zusammensetzung funktional und synergistisch zusammenwirken.

Das Gewichtsverhältnis von Chlorhexidin zu N-Cetylpyridiniumchlorid liegt zwischen 0 bis 7, insbesondere zwischen 0.01 bis 6, speziell zwischen 0.1 bis 5 im Speziellen zwischen 0.2 bis 4, beispielsweise zwischen 0.4 bis 3.2.

Falls Chlorhexidin vorhanden ist, liegt das Gewichtsverhältnis zwischen Chlorhexidin und N-Cetylpyridiniumchlorid zwischen >0 bis 7, insbesondere zwischen 0.01 bis 6, speziell zwischen 0.1 bis 5 im Speziellen zwischen 0.2 bis 4, beispielsweise zwischen 0.4 bis 3.2.

In einer besonderen Ausführungsform ist das Gewichtsverhältnis von Chlorhexidin zu N-Cetylpyridiniumchlorid < 0.1, im Besonderen < 0.01.

Da

Das erfindungsgemässe Gewichtsverhältnisses von Chlorhexiding zu N-Cetylpyridiniumchlorid führt zu einer besonders effektiven Hemmung von *S. Mutans* und einer besonders hohen Schonung des Speichelbioms.

Insbesondere beträgt ein Anteil an Chlorhexidin maximal 0.08 Gew. - %, insbesondere maximal 0.07 Gew. - %, besonders bevorzugt maximal 0.06 Gew. - %, im Speziellen maximal 0.05 Gew. - %, äusserst bevorzugt maximal 0.01 Gew. - %, bezogen auf das Gesamtgewicht der Zusammensetzung.

Durch die erfindungsgemässen Anteile von Chlorhexidin können Nebeneffekte von Chlorhexidin, wie z.B. eine Verfärbung der Zähne, Geschmacksstörungen, Mundtrockenheit, eine Veränderung der Mundflora, allergische Reaktionen oder ein erhöhter Blutdruck, reduziert werden.

In einer besonders bevorzugten Ausführungsform umfasst die erfindungsgemässe Zusammensetzung kein Chlorhexidin. In diesem Fall ist das Verhältnis von Chlorhexidin zu N-Cetylpyridiniumchlorid gleich Null.

Dies hat den Vorteil, dass die oben genannten unerwünschten Nebeneffekte von Chlorhexidin zumindest verringert, insbesondere ganz eliminiert werden können.

Ein weiterer Vorteil ist, dass durch die Reduzierung, insbesondere durch die Abwesenheit, von Chlorhexidin die Wirkung von CPC verstärkt werden kann.

Es hat sich deshalb herausgestellt, dass es durch die Reduzierung, insbesondere durch die Abwesenheit, von Chlorhexidin weniger von den anderen Bestandteilen, d.h. CPC, Flavonoide und/oder Polymere umfassend mindestens ein Monomer mit einer polaren funktionellen Gruppe braucht, ohne dass sich die Wirkung der Zusammensetzung verringert.

In einer besonderen Ausführungsform umfasst die erfindungsgemässe Zusammensetzung, insbesondere anstelle des mindestens einen Flavonoids, das orale Antiseptikum CITROX^{®}. CITROX^{®} umfasst u.a. Polylysin, Bioflavonoide, Zitronensäure, Glyzerin und Wasser.

Im Speziellen umfasst die erfindungsgemässe Zusammensetzung mindestens eine Fruchtsäure, insbesondere Zitronensäure, und/oder Glyzerin.

Im Speziellen beträgt ein Anteil an N-Cetylpyridiniumchlorid 0.005 bis 0.045 Gew. - %, insbesondere 0.015 bis 0.035 Gew. - %, im Speziellen 0.02 bis 0.03 Gew. - %, bezogen auf das Gesamtgewicht der Zusammensetzung.

Dies führt zu einer besonders effektiven Hemmung von S. *mutans* und einer besonders hohen Schonung des Speichelbioms.

Bevorzugt stammen die Flavonoide aus der Pflanze *Citrus aurantium amara.*

Diese Flavonoide haben die positiven Eigenschaften, dass sie entzündliche Prozesse hemmen und somit helfen, Entzündungen zu lindern. Dies ist besonders vorteilhaft bei der Prävention von Krankheiten wie Arthritis und anderen entzündungsbedingten Störungen ist.

Weiter können die Flavonoide aus der Bitterorange (*Citrus aurantium amara*) die Herzgesundheit unterstützen, indem sie den Blutdruck senken, die Blutfettwerte (z.B. Cholesterin) verbessern und die Endothelfunktion fördern. Die antioxidative und entzündungshemmende Wirkung dieser Flavonoide kann dazu beitragen, dass das Risiko von Herz-Kreislauf-Erkrankungen verringert wird.

Zudem können diese Flavonoide das Wachstum bestimmter Bakterien und Viren hemmen und so zur Stärkung des Immunsystems beitragen.

Es ist aber auch möglich, dass die Flavonoide aus anderen Pflanzen stammen, bspw. aus der Süssorange (*Citrus sinensis*)*,* Blau-, Him-, Erd- oder schwarzen Johannisbeeren, Äpfeln, Zwiebeln, Trauben, Wein, Sojabohnen, Buchweizen, Brokkoli, Kohl, Petersilie, Thymian, Ginkgo biloba, Rotklee, Chili und/oder Paprika.

Speziell bevorzugt sind die Flavonoide Flavanone, im Speziellen glycosylierte und/oder nicht-glycosylierte Flavanone.

Flavanone gehören zur Gruppe der Flavanoide, sind weit verbreitet in Zitrusfrüchten und haben aufgrund ihrer Struktur antioxidative und entzündungshemmende Eigenschaften.

Glycosylierte Flavanone sind Flavanone, die mit einem Zuckermolekül verbunden sind. Dadurch können sie eine bessere Wasserlöslichkeit aufweisen, was ihre Bioverfügbarkeit erhöhen kann.

Zudem können die Zuckermoleküle die Stabilität der Flavanone im Blut erhöhen, was ihre Halbwertszeit verlängern und allenfalls für eine verlängerte Wirkung im Körper führen kann.

Nicht-glycosylierte Flavanone haben keine Zuckermoleküle an sich gebunden. Dadurch können sie eine einfachere Struktur aufweisen, die zu einer einfacheren Aufnahme in die Zellen führen und ihre antioxidativen und entzündungshemmenden Eigenschaften verstärken kann. Dies kann insbesondere bei akuten Entzündungen hilfreich sein.

In einer weiteren bevorzugten Ausführungsform umfassen die Flavanone Naringin, Neohesperidin, Neoriocitrin, Isinarinagin, Hesperidin, Poncirin, Rhiofolin, Naringenin, Hesperetin und/oder Neodiosmin.

Diese Flavanone haben den Vorteil, dass sie eine besonders entzündungshemmende Wirkung aufweisen können.

Im Besonderen beträgt ein Anteil an Flavonoiden 0.02 bis 0.2 Gew. - %, insbesondere 0.04 bis 0.17 Gew. - %, im Speziellen 0.06 bis 0.15 Gew. - %, besonders bevorzugt 0.08 bis 0.12 Gew. - %, bezogen auf das Gesamtgewicht der Zusammensetzung.

Dies führt zu einer besonders effektiven Hemmung von S. *mutans* und einer besonders hohen Schonung des Speichelbioms.

In einer besonders bevorzugten Ausführungsform handelt es sich beim Polymer umfassend mindestens ein Monomer mit einer polaren funktionellen Gruppe um ein Homopolymer, im Speziellen um Polylysin.

Der Vorteil von Polylysin ist, dass es eine starke mikrobielle Wirkung aufweist, Plaque reduziert, Biofilme hemmt, als Konservierungsmittel dient, eine sanfte Wirkung auf das Zahnfleisch hat und die orale Gesundheit ohne Resistenzbildung fördern kann und dabei biologisch abbaubar und ungiftig für den Menschen ist.

Polylysin kann die Zellmembran von Mikroorganismen, wie z.B. S. *mutans,* destabilisieren und so zu einer Wachstumshemmung führen. Dadurch kann die Bildung von Plaque, Karies, Gingivitis, Halitosis und/oder Arteriosklerose zumindest minimiert werden.

Da Polylysin als Konservierungsmittel dienen kann, kann es das Wachstum von schädlichen Mikroorganismen in der Produktformulierung selbst hemmen und so zu einer längeren Haltbarkeit des Produkts beitragen.

Im Gegensatz zu Antibiotika kann Polylysin zudem die orale Gesundheit fördern, ohne das Risiko, dass die Bakterien im Mund resistent gegen die Behandlung werden.

Ausserdem weist Polylysin eine hohe Kationizität auf, was bedeutet, dass es positiv geladene Gruppen enthält, die sich gut an die negativ geladenen Oberflächen der Zähne und dem Zahnfleisch anlagern können. Diese elektrostatische Anziehung fördert die Haftung auf der Zahnoberfläche.

Insbesondere ist das mindestens eine Polymer umfassend mindestens ein Monomer mit einer polaren funktionellen Gruppe ε-Poly-L-Lysine.

ε-Poly-L-Lysine hat den Vorteil, dass es starke antimikrobielle Eigenschaften, eine hohe Hitzestabilität und Wasserlöslichkeit, eine gute biologische Abbaubarkeit aufweist und in den eingesetzten Mengen ungiftig ist.

Es ist aber auch möglich, dass andere Polymere, einzeln oder in Kombination mit Polylysin, eingesetzt werden, bspw. Polyvinylalkohol, Chitosan, Guar Hydroxypropyltrimonium Chlorid und/oder Carboxymethylcellulose.

Im Speziellen beträgt ein Anteil des mindestens einen Polymers umfassend mindestens ein Monomer mit einer polaren funktionellen Gruppe 0.002 bis 0.045 Gew. - %, insbesondere 0.015 bis 0.035 Gew. - %, im Speziellen 0.02 bis 0.03 Gew. - %, bezogen auf das Gesamtgewicht der Zusammensetzung.

In einer weiteren speziellen Ausführungsform umfasst die Zusammensetzung Lävulinsäure und/oder Salze der Lävulinsäure. Dies insbesondere zusätzlich zu den für Mundhygienemitteln gängigen Konservierungsmitteln, wie z.B. Sorbate und/oder Benzoate, im Besonderen jeweils einzeln oder in Kombination mit deren Säure(n).

Dies hat den Vorteil, dass die Wirksamkeit von Mundhygieneprodukten verbessert werden kann, da Lävulinsäure und/oder Salze der Lävulinsäure antimikrobiell wirken, entzündungshemmende Eigenschaften aufweisen, zur Wundheilung und Regeneration von oralem Gewebe oder zur pH-Regulierung beitragen und/oder als Konservierungsmittel oder Süssungsmittel eingesetzt werden können. Zudem weisen Lävulinsäure und/oder Salze der Lävulinsäure ein geringes Risiko für toxische Reaktionen auf.

Es ist aber auch möglich, dass weder Lävulinsäure noch Salze der Lävulinsäure in der erfindungsgemässen Zusammensetzung vorhanden sind, und im Besonderen nur konventionelle Konservierungsmitteln vorliegen.

Insbesondere beträgt ein Anteil an Lävulinsäure und/oder Salzen der Lävulinsäure 0.001 bis 1.5 Gew. - %, insbesondere 0.005 bis 1.3 Gew. - %, im Speziellen 0.010 bis 1.0 Gew. - %, bezogen auf das Gesamtgewicht der Zusammensetzung.

Besonders bevorzugt beträgt ein Anteil des mindestens einen Flavonoids, insbesondere aus der Pflanze *Citrus aurantium amara,* 0.02 bis 0.2 Gew. - %, ein Anteil des mindestens einen Polymers umfassend mindestens ein Monomer mit einer polaren funktionellen Gruppe, insbesondere Polylysin, im Speziellen ε-Poly-L-Lysine, 0.002 bis 0.045 Gew. - %, ein Anteil des N-Cetylpyridiniumchlorids 0.005 bis 0.045 Gew. - %, bezogen auf das Gesamtgewicht der Zusammensetzung, und es ist kein Chlorhexidin vorhanden.

Im Speziellen beträgt ein Anteil des mindestens einen Flavonoids, insbesondere aus der Pflanze *Citrus aurantium amara,* 0.1 Gew. - %, ein Anteil des mindestens einen Polymers umfassend mindestens ein Monomer mit einer polaren funktionellen Gruppe, insbesondere Polylysin, im Speziellen ε-Poly-L-Lysine, 0.025 Gew. - %, ein Anteil des N-Cetylpyridiniumchlorids 0.025 Gew. - %, bezogen auf das Gesamtgewicht der Zusammensetzung, und es ist kein Chlorhexidin vorhanden.

Dies führt zu einer besonders effektiven Hemmung von S. *mutans* und einer besonders hohen Schonung des Speichelbioms.

In einer anderen bevorzugten Ausführungsform umfasst die Zusammensetzung Magnolol und/oder ein Strukturisomer davon, wie z.B. Honokiol, und/oder ein Lignan, insbesondere ein lignanhaltiges Extrakt. In einer besonderen Ausführungsform handelt es sich um Magnolol.

Magnolol ist eine natürlich vorkommende chemische Verbindung und kommt als Hauptbestandteil in der Magnolienrinde vor.

Die Vorteile von Magnolol und Honokiol sind die antimikrobiellen Eigenschaften, insbesondere gegen Baktieren, die mit oralen Erkrankungen im Zusammenhang stehen, wie z.B. S. *mutans,* sowie seine entzündungshemmende und antioxidative Wirkung.

Es ist aber auch möglich, dass die Zusammensetzung kein Magnolol und/oder kein Strukturisomer davon und/oder kein Lignan umfasst.

Im Besonderen beträgt ein Anteil an Magnolol und/oder dem Strukturisomer davon, wie z.B. Honokiol, und/oder dem Lignan 0.005 bis 0.1 Gew. - %, insbesondere 0.006 bis 0.09 Gew. - %, im Speziellen 0.007 bis 0.08 Gew. - %, bezogen auf das Gesamtgewicht der Zusammensetzung.

Im Speziellen umfasst die Zusammensetzung Fluoride.

Fluoride haben den Vorteil, dass sie den Zahnschmelz stärken können, indem sie in die Mineralstruktur der Zähne integriert werden können. Dies macht die Zähne widerstandsfähiger und reduziert das Risiko von Karies. Weiter können Fluoride die Zähne stabilisieren und Mineralien wie z.B. Kalzium und Phosphat anziehen, womit frühe Karies behandelt werden kann. Zusätzlich weisen Fluoride eine antibakterielle Wirkung gegen Bakterien im Mund auf, wie z.B. gegen S. *mutans,* was der Reduzierung von Plaque dienen kann.

Es ist aber auch möglich, dass die erfindungsgemässe Zusammensetzung keine Fluoride umfasst.

Besonders speziell beträgt ein Anteil an Fluoriden 0.01 bis 0.2 Gew. - %, insbesondere 0.02 bis 0.1 Gew. - %, im Speziellen 0.03 bis 0.09 Gew. - %, äusserst speziell 0.04 bis 0.06 Gew. - %, bezogen auf das Gesamtgewicht der Zusammensetzung.

Insbesondere betrifft die vorliegende Erfindung Mundwasser, Zahnpasta, Zahnpflegekaugummis, Tabletten, Zahnseide und/oder Zahnreinigungsgel umfassend eine Zusammensetzung wie vorgehend beschrieben.

Dies vereinfacht die Anwendung der erfindungsgemässen Zusammensetzung, indem sie über die vorstehend genannten Mundhygieneprodukte dort aufgetragen werden kann, wo sie am effektivsten wirken kann, d.h. im Mundraum.

Es ist aber auch möglich, dass andere Produkte die erfindungsgemässe Zusammensetzung umfassen, bspw. Interdentalbürsten, Zungenreiniger und/oder Zahnaufhellungsprodukte.

Ein zweiter Aspekt der Erfindung beschäftigt sich mit einer Zusammensetzung wie vorhergehend beschrieben zur Verwendung bei der Prophylaxe und/oder Behandlung von Karies, Gingivitis, Halitosis und/oder Arteriosklerose.

In einem dritten Aspekt betrifft die Erfindung eine Zusammensetzung wie vorhergehend beschrieben zur Verwendung als Arzneimittel.

### Kurze Beschreibung der Zeichnungen

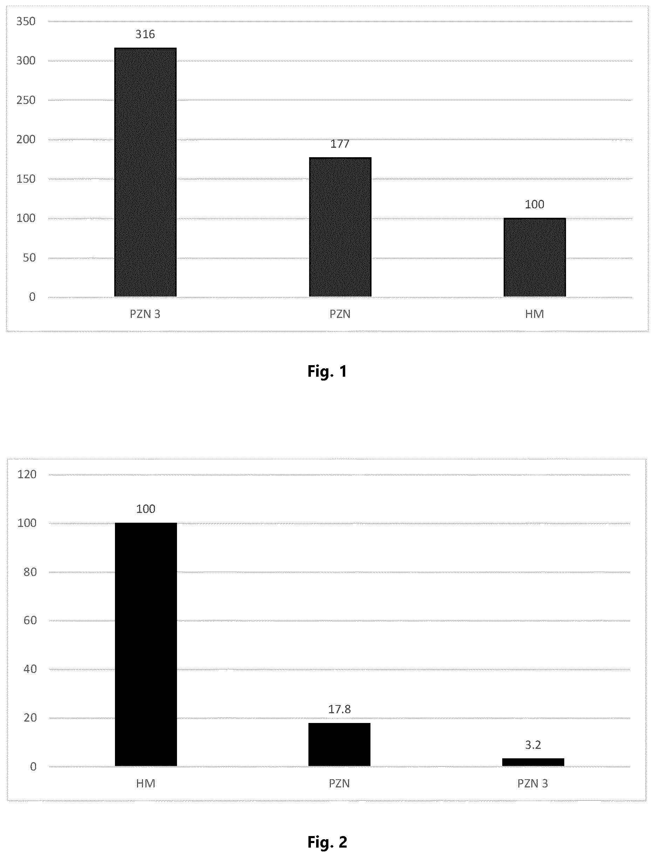

Fig. 1 Eine grafische Darstellung der minimalen Hemmkonzentration von zwei erfindungsgemässen Zusammensetzungen "PZN" und "PZN 3" sowie einer Zusammensetzung einer handelsüblichen Mundspülung "HM" in Bezug auf das Wachstum von *S*. *mutans.* Die minimale Hemmkonzentration ist dabei die höchste Verdünnung der Probe (1:Wert der vertikalen Achse) die das Wachstum vom *S. mutans* sichtbar hemmt.
Fig. 2 Eine grafische Darstellung der minimalen Hemmkonzentration von zwei erfindungsgemässen Zusammensetzungen "PZN" und "PZN 3" sowie einer Zusammensetzung handelsüblichen Mundspülung "HM" in Bezug auf die Beeinträchtigung des Speichelbioms. Die minimale Hemmkonzentration ist dabei die höchste Verdünnung der Probe (1:Wert der vertikalen Achse), die das Wachstum des Speichelbioms sichtbar beeinträchtigt.

### Experimente

Die Effekte der vorliegend beanspruchten Erfindung werden durch die folgenden experimentellen Daten gestützt.

Für drei unterschiedliche Zusammensetzungen "PZN", "PZN3" und "HM" wurde die Wirksamkeit der Hemmung von S. *mutans* sowie die Beeinträchtigung des Speichelbioms gemessen. Allen drei Zusammensetzungen liegen die nachfolgenden Basis-Bestandteile zu gleichen Teilen zugrunde:
Propylenglykol, Natriumgluconat, Xylit, Polyglyceryl-3-Caprat, Natriumhyaluronat, Polysorbat 20, Natriumchlorid, Natriumhydroxid, Trinatriumethylendiamindisukzinat, Cyclodextrin, VP/VA-Copolymer, Aroma Givaudan Top Noch, Sucralose, GuarHydroxypropyltrimoniumchlorid, Zitronensäure anhydr., Natriumphosphatbuffer und Natriumbenzoat.

"PZN", "PZN3" und "HM" unterscheiden sich in den variablen Bestandteilen gemäss der nachstehenden Tabelle.

| **Variable Bestandteile** | **PZN [Gew.** - %] | **PZN 3 [Gew.** - %] | **HM [Gew.** - %] |
|---|---|---|---|
| *Citrus Aurantium Amara* Fruchtextrakt (Flavonoide) | 0.100 | 0.100 | 0.010 |
| Magnolol | 0.075 | - | - |
| Polylysin (Homopolymer) | 0.025 | 0.025 | 0.005 |
| N-Cetylpyridiniumchlorid | 0.070 | 0.025 | 0.025 |
| Levulinsäure und Natriumlevulinat | 1.000 | - | - |
| Chlorhexidin | - | - | 0.200 |

Die Angaben in Gew. - % beziehen sich jeweils auf das Gesamtgewicht der jeweiligen Zusammensetzung.

Für die Verdünnung der Zusammensetzungen (jeweils Basis-Bestandteile + Variable Bestandteile) wurde für alle drei Zusammensetzungen Wasser als Lösungsmittel verwendet, und daraus eine Verdünnungsreihe hergestellt.

"PZN" und "PZN 3" entsprechen Ausführungsformen der vorliegenden Erfindung. "HM" entspricht einer Zusammensetzung einer handelsüblichen Mundspülung.

Figur 1 zeigt die Minimale Hemmkonzentration für die drei Zusammensetzungen "PZN", "PZN3" und "HM" in Bezug auf die Hemmung des Wachstums von S. *mutans.* "HM" entspricht dabei einer handelsüblichen chlorhexidinhaltigen Mundspülung, wie sie im Handel erhältlich ist.

Die minimale Hemmkonzentration bzw. die niedrigste Konzentration einer Verdünnungsreihe ist die niedrigste Konzentration eines antimikrobiellen Wirkstoffs, die erforderlich ist, um das Wachstum eines bestimmten Mikroorganismus unter standardisierten Bedingungen zu hemmen. Mit anderen Worten ist es die kleinste Menge eines Antibiotikums oder Antiseptikums, die benötigt wird, um die Vermehrung von Bakterien, Pilzen oder anderen Mikroorganismen zu verhindern.

Figur 1 zeigt auf der x-Achse die jeweiligen Zusammensetzungen "PZN", "PZN3" und "HM" und auf der y-Achse die Verdünnungswerte, mit welchen die oben beschriebenen Verdünnungslösungen jeweils zusätzlich mit Wasser im Verhältnis 1:"Verdünnungswert" weiter verdünnt wurden.

Aus Figur 1 ist ersichtlich, dass "PZN 3" bereits ab einer Verdünnung von 1:316 das Wachstum von *S*. *mutans* sichtlich beeinträchtigt, gefolgt von "PZN" bei einer Verdünnung von 1:177. Die marktübliche Zusammensetzung "HM" hingegen schneidet am schlechtesten ab und zeigt eine sichtbare Beeinträchtigung des Wachstums von S. *mutans* erst ab einer Verdünnung von 1:100. Damit hemmen die erfindungsgemässen Zusammensetzungen "PZN" und "PZN 3" das Wachstum von *S*. *mutans* bereits bei hohen Verdünnungswerten und gänzlich ohne Chlorhexidin, was heisst, dass im Vergleich zu "HM"" eine geringere Menge der jeweiligen Zusammensetzung nötig ist, um den gewünschten Effekt zu erzielen.

Figur 2 zeigt die Minimale Hemmkonzentration für die drei Zusammensetzungen "PZN", "PZN3" und "HM" in Bezug auf die Beeinträchtigung des Wachstums des Speichelbioms.

Figur 2 zeigt auf der x-Achse die jeweiligen Zusammensetzungen "PZN", "PZN3" und "HM" und auf der y-Achse die Verdünnungswerte, mit welchen die oben beschriebenen Verdünnungslösungen jeweils zusätzlich mit Wasser im Verhältnis 1:"Verdünnungswert" weiter verdünnt wurden.

Aus Figur 2 ist ersichtlich, dass "PZN 3" erst ab einer Verdünnung von 1:3.2 das Wachstum des Speichelbioms sichtlich beeinträchtigt, gefolgt von "PZN" bei einer Verdünnung von 1:17.8. Die marktübliche Zusammensetzung "HM" hingegen schneidet am schlechtesten ab und zeigt eine sichtbare Beeinträchtigung des Wachstums des Speichelbioms schon ab einer Verdünnung von 1:100. Damit hemmen die erfindungsgemässen Zusammensetzungen "PZN" und "PZN 3" erst ab niedrigen Verdünnungswerten, bzw. höheren Konzentrationen, das Wachstum des Speichelbioms, im Gegensatz zu "HM", welches bereits ab einem höheren Verdünnungswert, bzw. einer niedrigeren Konzentration, den unerwünschten Effekt herbeiführt, d.h. das Speichelbiom beeinträchtigt.

Die vorhergehend beschriebenen experimentellen Resultate legen die Schlussfolgerung nahe, dass die Abwesenheit von Chlorhexidin die Wirksamkeit der Zusammensetzung in Bezug auf die Verhinderung des Wachstum von *S*. *mutans* und die Schonung des Speichelbioms bzw. der Remanenz erhöht (vgl. "PZN" und "PZN 3" ohne Chlorhexidin mit "HM" inkl. Chlorhexidin).

Eine weitere Schlussfolgerung daraus ist, dass "PZN" und "PZN 3" selektiver auf schädliche Bakterien wie *S*. *mutans* wirken, als "HM".

## Patentansprüche

1. Eine Zusammensetzung, insbesondere zur Prophylaxe und/oder Behandlung von Karies, Gingivitis, Halitosis und/oder Arteriosklerose, umfassend:
(i) N-Cetylpyridiniumchlorid,
(ii) mindestens ein Flavonoid,
(iii) mindestens ein Polymer, im Besonderen ein Homopolymer, umfassend mindestens ein Monomer mit einer polaren funktionellen Gruppe, insbesondere Polylysin, und
(iv) optional Chlorhexidin,
(v) wobei ein Gewichtsverhältnis zwischen Chlorhexidin und N-Cetylpyridiniumchlorid zwischen 0 bis 7, insbesondere zwischen 0.01 bis 6, speziell zwischen 0.1 bis 5 im Speziellen zwischen 0.2 bis 4, beispielsweise zwischen 0.4 bis 3.2, liegt.

2. Die Zusammensetzung nach Anspruch 1, wobei ein Anteil an Chlorhexidin maximal 0.08 Gew. - %, insbesondere maximal 0.07 Gew. - %, besonders bevorzugt maximal 0.06 Gew. - %, im Speziellen maximal 0.05 Gew. - %, äusserst bevorzugt maximal 0.01 Gew. - %, bezogen auf das Gesamtgewicht der Zusammensetzung, beträgt.

3. Die Zusammensetzung nach einem der vorangehenden Ansprüche, wobei ein Anteil an N-Cetylpyridiniumchlorid 0.005 bis 0.045 Gew. - %, insbesondere 0.015 bis 0.035 Gew. - %, im Speziellen 0.02 bis 0.03 Gew. - %, bezogen auf das Gesamtgewicht der Zusammensetzung, beträgt.

4. Die Zusammensetzung nach einem der vorangehenden Ansprüche, wobei die Flavonoide aus der Pflanze *Citrus aurantium amara* stammen.

5. Die Zusammensetzung nach einem der vorangehenden Ansprüche, wobei die Flavonoide Flavanone, im Speziellen glycosylierte und/oder nicht-glycosylierte Flavanone, sind.

6. Die Zusammensetzung nach Anspruch 5, wobei die Flavanone Naringin, Neohesperidin, Neoriocitrin, Isinarinagin, Hesperidin, Poncirin, Rhiofolin, Naringenin, Hesperetin und/oder Neodiosmin umfassen.

7. Die Zusammensetzung nach einem der vorangehenden Ansprüche, wobei ein Anteil an Flavonoiden 0.02 bis 0.2 Gew. - %, insbesondere 0.04 bis 0.17 Gew. - %, im Speziellen 0.06 bis 0.15 Gew. - %, besonders bevorzugt 0.08 bis 0.12 Gew. - %, bezogen auf das Gesamtgewicht der Zusammensetzung, beträgt.

8. Die Zusammensetzung nach einem der vorangehenden Ansprüche, wobei das mindestens eine Polymer umfassend mindestens ein Monomer mit einer polaren funktionellen Gruppe ε-Poly-L-Lysine ist.

9. Die Zusammensetzung nach einem der vorangehenden Ansprüche, wobei ein Anteil des mindestens einen Polymers umfassend mindestens ein Monomer mit einer polaren funktionellen Gruppe 0.002 bis 0.045 Gew. - %, insbesondere 0.015 bis 0.035 Gew. - %, im Speziellen 0.02 bis 0.03 Gew. - %, bezogen auf das Gesamtgewicht der Zusammensetzung, beträgt.

10. Die Zusammensetzung nach einem der vorangehenden Ansprüche, wobei die Zusammensetzung Lävulinsäure und/oder Salze der Lävulinsäure umfasst.

11. Die Zusammensetzung nach einem der vorangehenden Ansprüche, wobei ein Anteil an Lävulinsäure und/oder Salzen der Lävulinsäure 0.001 bis 1.5 Gew. - %, insbesondere 0.005 bis 1.3 Gew. - %, im Speziellen 0.010 bis 1.0 Gew. - %, bezogen auf das Gesamtgewicht der Zusammensetzung, beträgt.

12. Die Zusammensetzung einem der vorangehenden Ansprüche, wobei ein Anteil des mindestens einen Flavonoids, insbesondere aus der Pflanze *Citrus aurantium amara,* 0.02 bis 0.2 Gew. - %, ein Anteil des mindestens einen Polymers umfassend mindestens ein Monomer mit einer polaren funktionellen Gruppe, insbesondere Polylysin, im Speziellen ε-Poly-L-Lysine, 0.002 bis 0.045 Gew. - %, ein Anteil des N-Cetylpyridiniumchlorids 0.005 bis 0.045 Gew. - %, bezogen auf das Gesamtgewicht der Zusammensetzung, beträgt und kein Chlorhexidin vorhanden ist.

13. Die Zusammensetzung nach einem der vorangehenden Ansprüche, wobei die Zusammensetzung Magnolol und/oder ein Strukturisomer davon, wie z.B. Honokiol, und/oder ein Lignan, insbesondere ein lignanhaltiges Extrakt, enthält.

14. Mundwasser, Zahnpasta, Zahnpflegekaugummis, Tabletten, Zahnseide und/oder Zahnreinigungsgel umfassend eine Zusammensetzung nach einem der Ansprüche 1 - 13.

15. Zusammensetzung nach einem der Ansprüche 1 - 13 zur Verwendung bei der Prophylaxe und/oder Behandlung von Karies Gingivitis, Halitosis und/oder Arteriosklerose.

16. Zusammensetzung nach einem der Ansprüche 1 - 13 zur Verwendung als Arzneimittel.
